# EUROPEAN PATENT APPLICATION

(11) **EP 3 909 574 A1**
(43) Date of publication of application: **17.11.2021**
(21) Application number: 19908967.3
(22) Date of filing: 16.12.2019
(51) Int. Cl.: A61K 31/196, A61K 31/573, A61P 19/02

(54) **SYNERGIC PHARMACEUTICAL COMPOSITION OF ACECLOFENAC AND BETAMETHASONE FOR TREATING THE PAIN OF RHEUMATIC CONDITIONS OR POSTSURGICAL PAIN**

(30) Priority: 08.01.2019 MX 2019000312
(71) Applicant: Amézcua Amézcua, Carlos, Guadalajara, Jalisco, 44898 (MX)
(72) Inventor: GARCÍA ARMENTA, Patricia del Carmen, Jalisco, 45019 (MX); CHAVEZ GARCÍA, José Alonso, Jalisco, 45138 (MX); AMEZCUA AMEZCUA, Federico, Jalisco, 44898 (MX)
(74) Representative: Ungria López, Javier
(86) International application number: PCT/MX2019/000139
(87) International publication number: WO 2020/145811

(57) **Abstract**

The present invention relates to a pharmaceutical composition formed by the synergic combination of a non-steroidal anti-inflammatory analgesic such as aceclofenac or its pharmaceutically acceptable salts, and a steroidal anti-inflammatory such as the active ingredient betamethasone or its pharmaceutically acceptable salts of phosphate or dipropionate, which are formulated to be administered via intramuscular or intravenous injection, for the treatment of localized pain in rheumatic conditions or in postsurgical patients.

## Description

### FIELD OF THE INVENTION

The present invention relates to the technical field of the pharmaceutical industry, and its object is to provide a pharmaceutical composition comprising the synergic pharmaceutical combination of a non-steroidal anti-inflammatory pharmaceutical compound (NSAID), constituted by the active ingredient aceclofenac or pharmaceutically acceptable salts thereof and a steroidal anti-inflammatory pharmaceutical compound from the group of corticosteroids such as the active ingredient betamethasone or pharmaceutically acceptable salts thereof selected from phosphates or dipropionates, as well as pharmaceutically acceptable carriers, excipients or adjuvants, formulated as a solution to be administered as intramuscular or intravenous injection. Said combination is indicated for the control and treatment of pain caused by rheumatic diseases or post-surgical pain.

The combination of the aforementioned active principles shows improved properties and provides a greater therapeutic effect if said active principles are jointly administered as a single dosing unit instead of being administered separately, providing benefits such as lower administered doses, greater therapeutic effect and less adverse effects.

### BACKGROUND OF THE INVENTION

Pain is an unpleasant sensory and emotional experience and as such is personal. Although it cannot be transferred, it can be expressed. This experience is unique, unrepeatable and transcendent, encompassing neurophysiological, psychological, behavioral and cultural mechanisms. Thusly defined, pain is a multidimensional, psychophysical and sociocultural problem with far-reaching implications that surpass patients, their families and even their societies.

Several definitions of pain have been proposed, although it has been considered a sensory response to tissue injury only since 1960. However, in those days there was little information available on the affective component of pain. In 1979 the International Association for the Study of Pain (IASP) defined it as "An unpleasant sensory and emotional experience associated with actual or potential tissue damage, or Disclosed in terms of such damage".

This definition of pain is still considered as the most adequate, since it differs from past definitions by introducing two new concepts:
a) It considers pain not as a mere sensory response to tissue damage, but also encompassing emotional and subjective components.
b) It can be suffered without being justified by a bodily cause.

### NOCICEPTION

Pain starts in special receptors or nociceptors, which are disseminated in the whole body. These receptors transfer information as electric impulses sent through nerves to the spinal cord and then to the brain. Occasionally these signals cause a reflex action when reaching the spinal cord and, if this happens, the signals are immediately re-sent by motor nerves to the origin point of pain, causing muscular contractions.

The signal also reaches the brain, where it is processed and interpreted as pain, and is noticed by conscience of the individual.

Pain can be classified in several ways, for example by its time of evolution, as acute or chronic; by its origin, as neoplastic or non-neoplastic, among others. This classification is related to the etiology, pathophysiology and symptomatology of the pain. However, the most important aspects are related to diagnosis and therapy.

Presently there are many health problems greatly relevant for Public Health due to their magnitude and importance. In this sense, there is a set of different diseases having in common that they affect the musculoskeletal system and are seldom mentioned among health professionals, since it is generally thought that they are unimportant for daily life, or affect only elderly adults.

Musculoskeletal system diseases are known as rheumatic diseases and are more frequent than is generally thought. In Mexico, rheumatic diseases are the third leading cause for seeking help from general practitioners, and account for 16% of all medical consultations. According to the National Institute of Statistics and Geography of Mexico, in 1995 the country had 93 million inhabitants and rheumatic diseases were the first cause of permanent disability. It is estimated that in Mexico there are 270,000 to 900,000 patients with rheumatoid arthritis and about 6 million persons with degenerative joint diseases. Thus, rheumatic diseases have a serious impact on public health, and could be even greater when taking in account other common health problems such as obesity, which in Mexico has reached epidemic levels.

Rheumatic diseases are among the leading causes of disability. The severity of these diseases is variable, from mild pain in the joints and associated structures (muscles, tendons and ligaments) to life threatening disorders. Rheumatology deals with a broad spectrum of diseases, such as: rheumatoid arthritis; osteoarthritis; ankylosing spondylitis; bursitis; tendinitis; synovitis; lower back pain (lumbago); crystalline arthropathies (i.e., gout); psoriatic arthritis.

Rheumatoid arthritis (RA) is a systemic, chronic, inflammatory and autoimmune disease, affecting connective tissues and several joints. It is a progressive and highly disabling pathology and more prevalent in female patients.

It has been estimated that about 10% of the global population develops some rheumatic disease during lifetime. According to the 1998 World Health Report of the World Health Organization, about 190 million people suffer from degenerative joint diseases, and 16.5 million suffer from rheumatoid arthritis. According to estimations, the global prevalence of rheumathoid arthritis is about 1% (0.3-2.1%), and 15% of males and 25% of females over 60 years old have symptomatic degenerative joint diseases.

The most common clinical symptoms are arthralgias, morning stiffness, tiredness, weight loss and feverishness. Carpal tunnel syndrome may also be observed in the feet and metatarsophalangeal joints.

Osteoarthritis, the most common form of arthritis, is a chronic degenerative joint disease that affects mainly middle aged and elderly adults.

The main characteristic of osteoarthritis is breakdown and eventual loss of joint cartilages; it can affect the neck adjacent bones, lower back, knees and hand fingers.

This arthropathy is characterized by cartilage degeneration and joint margins hypertrophy, usually with minimal inflammation.

There are two main types of osteoarthritis:
PRIMARY: Affects some or all distal interphalangeal joints (Heberden's nodes), and less commonly the proximal interphalangeal joints (Boudchard's nodes), the carpometacarpal and metacarpophalangeal joints of the thumb, and also the hips, knees, metatarsophalangeal joints of the first toe, and cervical and lumbar vertebrae.

SECONDARY: It can develop in any joint as a sequel of lesions of extraarticular and intraarticular origin, including rheumatoid arthritis.

Lesions can be acute such as fractures, chronic due to occupational overuse of a joint, or due to metabolic diseases, i.e., hyperparathyroidism, hemochromatosis or ochronosis.

As a secondary incidence, obesity may be a significant risk factor for the development of osteoarthritis in the knees and probably in the hips. The most frequent symptom of osteoarthritis is pain after joint overuse or long periods of inactivity. The pain begins insidiously, initially with articular stiffness rarely lasting more than 15 minutes, and then there is pain upon movement of the affected joint which worsens if there is activity or when lifting heavy loads. Rest alleviates the pain. Deformities may be minimal or absent. However, the interphalangeal joint bones grow notoriously; flexion contractures and varus knees are frequent. There is no ankylosis, although frequently the movement of the affected joints becomes increasingly limited. It is common to feel a coarse crepitation in the affected joint; however, joint effusions and other signs of inflammation are mild.

Ankylopoietic or ankylosing spondylitis (AS) is a rheumatic disease that causes inflammation in vertebrae and sacroiliac joints. Its most common symptoms are lower back pain which can affect the whole spinal column and peripheral joints, causing spinal stiffness, mobility loss, progressive joint deformities and pain. It can be associated with extraarticular symptoms such as ocular and heart valves inflammation.

It is usually manifested in adolescents and young adults and is more prevalent in males. On the other hand, the disease is usually milder in females, making its diagnosis more difficult. Its incidence varies according to ethnicity.

Early manifestations are nocturnal pain and mobility loss in the lower back. Although symptoms usually begin in lumbar vertebrae and sacroiliac joints, they can also be observed in the cervical and dorsal segments of the spinal column.

Lower back pain is one of the most frequent reasons for seeking help from general practitioners and pain clinics.

According to estimations, about 4% of the population suffers from lower back pain every year, and more than 70% suffer at least one lower back pain (LBP) episode during lifetime.

Lumbago (LBP) is defined as a painful sensation located in the lumbar vertebrae that prevents normal mobility. LBP is classified as acute if it lasts less than 3 months, and as chronic if it lasts longer and is associated to effort intolerance, with or without affecting the lower limbs.

Mechanical symptoms are more frequent (90%) in LBP, triggered when moving the column, and subsiding with rest. However, inflammatory symptoms are continual, intense and persistent, and do not subside when resting. If the pain is insidious, constant, intense and agonizing, increases during rest and disturbs sleep, it is more characteristic of neoplastic pain.

Gout arthritis is a metabolism disorder of purines derived from proteins. If purines are overproduced and they build up without being adequately canalized, uric acid levels increase. Since uric acid is not deposited in the blood vessels it always reaches the kidneys -causing uric acid nephropathy and leading to kidney stones or urinary crystals- or joints, causing what is known as gout arthritis.

After repeated attacks, articular inflammation can become chronic and deforming. About 20% of gout patients develop kidney stones.

Bursitis and/or tendinitis is the inflammation of tendons (bands that connect muscles to bones); bursitis is the inflammation of one or more bursae (fluid-filled sacs which facilitate the normal movement of tendons and muscles over the bones). Tendons and bursae are located next to joints, and when inflamed they cause joint pain.

Symptoms are pain and inability of movement in the joint located next to the affected tendon or bursae. The most affected areas are elbows, shoulders, feet, ankles, knees, hips, wrists and fingers.

Inflammation is caused by overload in the affected area (injuries); thus, when the acute phase subsides there are no residual lesions.

Psoriatic arthritis (PA) is a joint disorder appearing in 10-20% of skin psoriasis patients, and its prognosis and evolution has peculiar characteristics. PA joint lesions are inflammatory, i.e., associated with pain, swelling, heat, and impaired movement of the inflamed joint which in the long term may be deformed.

It is a chronic disease that evolves irregularly during lifetime, with alternating periods of inactivity and times with inflammation and pain.

The disease is manifested differently in each individual, although there are five main types:
1. Asymmetric oligoarticular arthritis, affecting only few joints in the limbs, and is the most common type.
2. Arthritis mainly affecting the hands' distal interphalangeal joints.
3. Symmetric arthritis similar to rheumatoid arthritis.
4. Arthritis mutilans, a rare although destructive and crippling type.
5. Arthritis affecting vertebrae and pelvic or sacroiliac joints, similarly to ankylosing spondylitis, another rheumatic disease.

The onset of psoriatic arthritis usually occurs between 30 and 50 years of age in both genders, although it can appear at any age and even in children.

Articular manifestations are common to all types of arthritis: Pain, heat, redness, inability to move the joint and occasionally deformation. Any joint can be affected, including jaws and toes (which coincidentally are quite common). If the inflammation is located in the spine, particularly in the sacroiliac joints, one of the most frequent symptoms is nocturnal pain in the buttocks, which awakes the patient during the early morning and after having slept 4 or 5 hours. Pain in the heels when waking up and walking the first steps is also common, as well as stabbing pain in the chest when breathing deeply. Another articular manifestation is stiffness lasting more than 30 minutes when waking up. Opening and closing hands becomes difficult; upon moving the joint it feels like "rusted" and even holding a toothbrush becomes uneasy.

Joint diseases affect about 2-3% of the global population, and 25-34% of these patients suffer from psoriatic arthritis. More than 80% of patients suffer the skin disease first, and on average psoriatic arthritis sets in 10 years after the first symptoms of skin psoriasis.

Pain is highly prevalent and significantly impacts on individuals, their families and jobs, as well as on the society and economy.

It has been scientifically observed that a high overweight and obesity prevalence in subjects with knee arthritis worsens the clinical picture, causing more pain and functional disability.

Also, the metabolic syndrome is a clearly important cardiovascular risk factor, and according to several studies has a high prevalence in some autoimmune rheumatic diseases; it has been found in 35% of ankylosing spondylitis patients and 36.2% of primary antiphospholipid syndrome patients.

Rheumatic diseases are not limited to the adult population, and among those diseases that can set in during childhood are juvenile rheumatoid arthritis and juvenile spondyloarthropathies; both appear at an early age and if not treated promptly have serious consequences such as joint deformities, functional inability, several degrees of disability, impairment of quality of life and social performance.

It is interesting to note that rheumatic diseases are generally more common than other pathologies better known by the population. Since pain is the main manifestation of these diseases, it is very likely that the patient will self-medicate with commonly used analgesics and both steroidal and non-steroidal anti-inflammatory pharmaceutical compounds before seeking for specialized health care. There may be complications if the primary care physician misdiagnoses the disease, or if the referral to a specialist is inadequate. A delayed treatment will most likely worsen the patient's functional prognosis, resulting in high rates of temporary incapacity and disability, loss of productivity costs, quality of life's impairment and even premature death.

Thus, a successful strategy is combining active ingredients that are both easily available and effective and whose mechanisms of action complement each other, in order to improve their clinical safety and to obtain an improved therapeutic benefit.

The group of non-steroidal anti-inflammatory pharmaceutical compound is one of the most prescribed globally for the management and treatment of pain-related diseases. NSAIDS have an analgesic effect useful for rheumatic pain, both in inflammatory and degenerative diseases, and are also often used in non-rheumatic diseases such as migraine, dental pain and in other processes involving pain. Moreover, these active ingredients are useful as antipyretics. It should be noted that recently it has been proved a colon cancer prevention effect of NSAIDS. The use of these pharmaceutical compounds is widespread in the general population, even as self-medication, since they are available over the counter, with the potential risk of side-effects.

Some examples of non-steroidal anti-inflammatory active ingredients are acetylsalicylic acid, salsalate, diflunisal, phosphasal, lysine acetylsalicylate, phenylbutazone, indometacin, tolmetin, sulindac, acemetacin, diclofenac, aceclofenac, nabumetone, ibuprofen, naproxen, ketoprofen, flurbiprofen, piroxicam, tenoxicam, meloxicam, mefenamic acid, meclofenamate, celecoxib, etoricoxib and lumiracoxib.

Of the above-mentioned active ingredients, aceclofenac has analgesic and antipyretic properties. At an experimental level, it inhibits oedema and erythema formation, regardless of the inflammation etiology. The study of its mechanism of action both in humans and animals has shown that aceclofenac inhibits prostaglandin and leukotriene formation through a reversible inhibition of cyclooxygenase,

The chemical name of aceclofenac is: 2-[2-[2-(2,6-dichlorophenyl) amino]phenyl]acetyl]oxyacetic acid , represented by the following molecule (I): 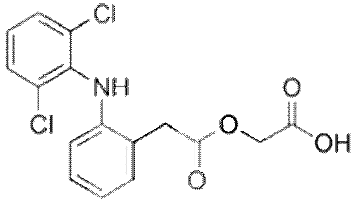 disclosed for the first time in US Patent No. 4,548,952, with anti-inflammatory and anesthetic properties.

Another group of anti-inflammatory active ingredients are corticosteroids, which also have immunosuppressive properties. Corticosteroids have been for a long time widely used by many specialists, because of their high efficiency and well-known benefits in many clinical situations. However, it should be noted that many uses of corticosteroids are empirical, and their mechanism of action, effective doses and clinical efficacy has not been properly studied. Some examples of corticosteroid active ingredients are hydrocortisone or cortisol, cortisone, prednisone, methylprednisolone, deflazacort, fludrocortisone, triamcinolone, paramethasone, betamethasone and dexamethasone.

Corticosteroids can be administered by several routes and is particularly important to know the advantages and limitations of each route. An ill-chosen route may result in more side effects, greater costs and less therapeutic efficiency. In some cases, a local administration is desired, while in others a systemic effect is preferred.

Corticoids share a common skeletal structure, having cyclopentanoperhydrophenanthrene as a nucleus with 17 carbon atoms arranged in 4 rings. Certain modifications increase their potency, such as double bonds in C1-C2, halogenation in C6 or C9, or the addition of hydroxyl groups or carbon chains such as acetonides, valerates and propionates.

The modifications in this structure enable the production of compounds with various degrees of potency and toxicity.

Its mechanism of action is intracellular: once inside the cytoplasm certain intracytoplasmic receptors transport these compounds to the nucleus, where they bind to specific DNA sequences - glucocorticoid response elements, thus inhibiting or stimulating the adjacent genetic transcription and regulating inflammatory processes.

To be useful for a topical route, adequate epidermic concentrations must be achieved, without inducing high serum levels. After its administration it creates a reservoir in the skin, and hence theoretically it is not necessary to administer it more than once daily or every two days.

The anti-inflammatory potency of a topical corticoid is measured by its capability of causing vasoconstriction in the skin; this capability is used to construct a scale of its relative potency (Stoughton assay). Hence, its efficiency is related to its potency, and so are its side effects. Generally, fluorinated derivates (betamethasone, fluocinolone, etc.) are more potent than non-fluorinated.

The chemical name of betamethasone is: (11β,16β)-9-Fluoro-11,17,21-trihydroxy-16-methylpregna-1,4-diene-3,20-dione, represented by the following molecule (II):

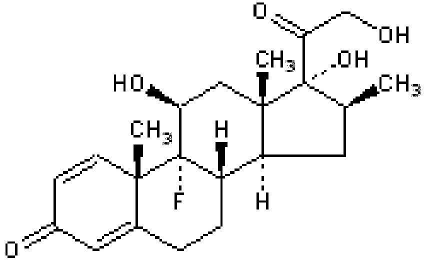

Disclosed for the first time in US Patent No. 3,053,865, which discloses its anti-inflammatory properties and its effects in the treatment of arthritis; US Patent No. 3,104,246 discloses more specifically its preparation process.

Betamethasone reduces inflammation by inhibiting the release of leukocytes hydrolases, preventing macrophage build-up at the site of inflammation. Betamethasone administration interferes with the leukocyte adhesion to capillary walls and reduces the capillary membrane permeability, which in turn reduces edema formation. Moreover, betamethasone downregulates inflammation promoting molecules such as histamines and quinines (IL-1, IL-6, TNFα), and inteferes with the formation of fibrous tissue.

Anti-inflammatory effects of corticoids are generally due to their effects on lipocortins, phospholipase A2-inhibiting proteins. Lipocortins control the synthesis of potent inflammation mediators such as leukotrienes and prostaglandins, since they inhibit the synthesis of their precursor arachidonic acid:
(9-Fluoro-11 ,1 7-dihydroxy-1 7-(2-hydroxyacetyl)-1 0,13,16- trimethyl-6,7,8,9,10,11,12,13,14,15, 16, 17-dodecahydrocyclopenta[a]phenanthren-3-one)

Betamethasone is a compound with high glucocorticoid but low mineralocorticoid activities; the anti-inflammatory activity of 0.75 mg of betamethasone is equivalent to 5 mg of prednisolone. Betamethasone dipropionate is the 17,21-dipropionate ester of betamethasone.

Betamethasone is a whitish crystalline odorless powder insoluble in water. It is rapidly absorbed in the GI tract although it is also absorbed by the skin. It distributes to all tissues in the body, binds to plasma proteins, mostly to globulins, has an extended half-life of 72 hours with an equivalent dose of 0.75 mg, and is metabolized in the liver and kidneys. It is excreted in urine. Betamethasone has fewer effects on sodium and water than prednisolone or prednisone.

An effective treatment is required in order to solve an anti-inflammatory problem. Said treatment most provide the needed effect for this problem, at lower doses than those commonly used, with a shorter time of action onset and fewer adverse effects. The present invention comprises an aceclofenac and betamethasone combination for the treatment of rheumatic pain and post-surgical pain.

The potential combinations of two or more pharmaceutical compounds are supported by clinical and experimental data. These combinations have shown analgesic efficiency, with less adverse effects than the drugs when administered separately. The combination of pharmaceutical compounds acting with different pharmaceutical mechanisms of action can provide potential benefits such as:
- Increasing the analgesic efficacy through a synergic or additive interaction.
- Extending the analgesic spectrum: opioids decrease the selectivity to stimuli unrelated to pain, and adjuvant pharmaceutical compounds diminish the selectivity to stimuli induced by pain.

Hence, the combination allows to:
- Decreasing the required doses of pharmaceutical compounds (opioids, NSAIDs, muscle relaxants, et cetera.)
- Diminishing adverse effects.

Given the number of pathways involved in the perception of pain, the World Health Organization and the American Pain Society have recommended therapeutic combinations. If doses are decreased, patients have a better tolerance to analgesics.

When two pharmaceutical compounds are jointly administered, their effects can be:
a) Additive, i.e., the sum of the effects delivered by the pharmaceutical compounds when administered separately.
b) Subadditive, also known as antagonistic; an effect lower than the sum of the pharmaceutical compounds when administered separately.
c) Synergic or supra-additive; an effect greater than than the sum of the drugs when administered separately.
d) Potentiation. The result of using an analgesic with a non-analgesic drug, and this combination results in a greater effect than that observed when using only an analgesic.

Evidently, the combination of synergism or potentiation-producing pharmaceutical compounds is a more promising treatment of pain. When considering that synergism is usually associated with significantly lower doses and less adverse reactions, the research of pharmaceutical compounds administered jointly and resulting in a synergic interaction becomes particularly important in pharmacology.

In state of the art, US Patents 7,070,765 and US 7,078,019 disclose an aerosol and administration method for delivery of a drug ester such as aceclofenac and betamethasone among others, characterized by less than 10% drug ester degradation products by weight, and an MMAD of less than 5 microns; US Patent 8,361,492 discloses a drug delivery system comprising: a contact lens comprising electrospun fibers incorporated into a polymer lens; wherein the electrospun fibers are prepared by electrospinning a polymer solution into a mat of fibers, applying a cross-linking treatment to the mat of fibers, and applying a polymer coating to the mat of fibers; wherein the at least one therapeutic drug selected from aceclofenac, betamethasone and other compounds is incorporated into the mat of fibers; US Patent 9,597,527 discloses a dermal system in the form of transdermal patch comprising at least one light source, wherein the light source emits infrared irradiation with a maximum emission in the range between 700 nm and 3 mm, the light source selected from organic light emitting diodes, polymeric light emitting diodes and at least one pharmaceutically and/or cosmetically active ingredient selected from aceclofenac and betamethasone for the treatment and/or prophylaxis of acute and chronic pain, muscle pain, joint stiffness, muscular tension and stiffness, mood disorders, menopause, osteoporosis, angina, acute injuries, arthritis, nicotin addiction, viral infections, inflammation, tumors, and cancer; Mexican Patent MX 348595 discloses a solid triphasic release system, which may be delayed release and/or extended release and/or modified release and/or immediate release, of at least three layers for the formation of at least one dosage unit, wherein each layer comprises, as active pharmaceutical ingredients, at least one corticosteroid agent of the betamethasone type and/or the pharmaceutically acceptable salts thereof, at least one non-steroidal antiinflammatory agent of the aceclofenac type and/or the pharmaceutically acceptable salts thereof, and at least one pharmaceutically acceptable excipient, for the treatment of inflammation and body pain.

The present invention is characterized by providing a composition comprising the combination of aceclofenac and betamethasone in semi-solid or liquid form, not previously reported in the state of the art. The potential advantage of therapies with this combination is that the analgesic effects may be maximized, while minimizing the incidence of adverse effects.

The use of this pharmaceutical compound combination offers a synergism, thus reducing the required doses and minimizing adverse effects.

### OBJECTIVE OF THE INVENTION

To provide a new therapeutic option for the control and treatment of rheumatic pain and post-surgical pain, capable of reducing symptoms and improving the patients' quality of life. This is accomplished through the strategy of combining aceclofenac or pharmaceutically acceptable salts thereof and betamethasone or pharmaceutically acceptable phosphate or dipropionate salts thereof; said combination results in a synergic interaction which increases its therapeutic potency and onset of action, while reducing adverse events.

Said combination improves therapy by offering benefits such as greater efficiency and therapeutic potency when being administered, with the aim of accomplishing local therapeutic effects for the treatment and management of pain caused by rheumatic diseases and post-surgical pain, while significantly reducing the likelihood of side effects.

### BRIEF DESCRIPTION OF THE DRAWINGS

Figure 1. Temporal course of formalin (average 6 rats).
Figure 2. Effect of aceclofenac intramuscular injection on formalin-induced pain behavior.
Figure 3. Effect of betamethasone intramuscular injection on formalin-induced pain behavior.
Figure 4. Effect of intramuscular coadministration of the aceclofenac-betamethasone mixture on formalin-induced pain behavior.
Figure 5. Isobologram of aceclofenac with betamethasone.
Figure 6. Dose-response curves (DRC) of aceclofenac combined with betamethasone 0.05 mg/Kg i.m. when graphing global antinociceptive effects as AUC's of temporal courses versus administered doses of aceclofenac. Graph points are expressed as mean ± standard error.
Figure 7. Dose-response curves (DRC) of aceclofenac combined with betamethasone 0.5 mg/Kg i.m. when graphing global antinociceptive effects as AUC's of temporal courses versus administered doses of aceclofenac. The graph points are expressed as mean ± standard error.

### DETAILED DESCRIPTION OF THE INVENTION

The present invention refers to the novel injectable pharmaceutical composition comprising at least one non-steroid anti-inflammatory pharmaceutical compound such as aceclofenac or pharmaceutically acceptable salts thereof and at least one corticosteroid agent such as betamethasone or pharmaceutically acceptable phosphate salts or dipropionate salts thereof, wherein said pharmaceutically active ingredients provide synergic effects when administered, and achieve local therapeutic effects in the treatment and control of rheumatic diseases pain and post-surgical pain.

The aceclofenac and betamethasone synergic formulation can be injectable either intramuscularly or intravenously to maximize its systemic distribution, providing a fast and effective local analgesic and anti-inflammatory effect.

The aceclofenac and betamethasone combination is an effective therapeutic resource in patients suffering from pain caused by rheumatic diseases or by post-surgical procedures, with minimal or no adverse events, and a significant local analgesic and anti-inflammatory action. It is considered for treating post-surgical pain in patients, and for alleviating or avoiding gastric pain caused by said active ingredients.

The concomitant use of glucocorticoids such as betamethasone with non-steroidal anti-inflammatories (aceclofenac) results in a therapeutic additive effect that has a favorable impact on populations.

The present invention has been tested in preclinical assays, and it has proved that the novel aceclofenac and betamethasone injectable combination provides a strong synergic therapeutic effect in the treatment of systemic or local rheumatic pain, and thus the present invention has as its main objective the development of a pharmaceutical composition comprising the combination of a non-steroid anti-inflammatory pharmaceutical compound such as aceclofenac and the corticosteroid agent such as betamethasone or pharmaceutically acceptable phosphate or dipropionate salts thereof. Said combination is formulated with pharmaceutically acceptable excipients and is indicated for the treatment and control of local rheumatic pain and for post-surgery patients.

Nowadays, an alternative to increase the efficacy of an analgesic treatment and to significantly reduce the secondary effects is through the combined administration of two or more active agents, such as the synergic pharmaceutical combination intended to be protected by the present invention.

The present invention intends to offer a new therapeutic option for the treatment and control of rheumatic and post-surgical pain, capable of reducing symptoms and improving the patients' quality of life.

There is presently no assessment of the effects provided by the aceclofenac and betamethasone combination injectable either intramuscularly or intravenously. Accordingly, this research evaluated the antinociceptive effects of the aceclofenac and betamethasone combination by means of an isobolographic analysis of the formalin-induced pain in the rat experimental model.

### Method

### Experiment animals

Male Wistar rats aged 7-8 weeks weighting 175-199 g were used in the experiments. Rats had free access to food and water before the experiments. All experimental procedures complied with the Guidelines on Ethical Standards for Investigations of Experimental Pain in Animals (IASP, 1983). Each rat was used only once and was sacrificed in a CO2-saturated glass bell.

The rats were kept in a room with alternating light/darkness cycles. All experiments were performed during the light phase of the light/darkness cycles.

### Formalin test

The formalin model is used mainly with rodents. The test consists in the subcutaneous administration of formalin (formaldehyde in concentrations ranging from 1 to 5%), in a 20-25 µL volume for mice 30-100 µL and for rats. The injection is usually administered in the dorsal surface of the right hind paw, and animals are sacrificed once the test is finished. The formalin test describes several behaviors, such as biting, licking and shaking the injected hind paw. Hind paw shaking is the most used parameter for quantifying the degree of pain.

The formalin test in rodents is biphasic. The first phase (acute or neurogenic) onsets after the injection and lasts 3-5 minutes, and it is caused by the direct stimulation of mainly C-fiber nociceptors. The second phase (tonic or inflammatory) onsets 15-20 minutes after the injection and lasts 20-40 minutes.

### Formalin-induced pain behavior

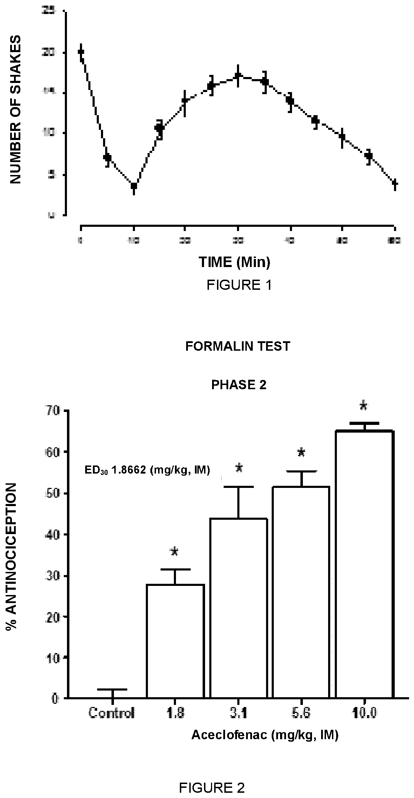

The 1%-formalin injection induced the typical shaking behavior, characterized by a brisk withdrawal or bending of the injected hind paw. The number of shakes was plotted vs. time, and graphs such as the one shown in Figure 1 were obtained. The temporal course was assessed in this figure after the 1%-formalin injection, and the biphasic (phase 1 and 2) behavior characterizing this model was observed.

### Isobologram analysis

An isobologram analysis was employed to assess aceclofenac and betamethasone interactions. An isobologram is a graphic method constructed by placing in both axes the ED's (for example ED30) of each pharmaceutical compound, in this case aceclofenac and betamethasone. When connecting the dots an additivity line is obtained, useful to establish the type of interaction of both compounds under study. Dose-response curves (DRC) are constructed to obtain the ED30's of each active principle. At least 4 doses of each active principle are administered by IM injection to at least 6 animals. The dose delivering 30% of the maximal effect (ED30) was calculated by means of a linear regression analysis based on the least squares of the DRC. The interactions of the different active principles were assessed by IM co-administration of aceclofenac and betamethasone. Before the test, the pharmaceutical compounds were diluted and mixed for intramuscular administration. Using a linear regression analysis of the log dose-reponse curves, the dose delivering 30% of the maximal effect (ED30) was determined for each pharmaceutical compounds mixture. This dose was statistically compared to the dose that theoretically is the simple addition of effects, and which can be obtained with the following formula: ED30 theoretical additivity = ED30 pharmaceutical compound 1/(P1 + R x P2)
where R is the potency ratio of pharmaceutical compounds 1 and 2 administered alone; P1 is the proportion of pharmaceutical compound 1 in the mixture; P2 is the proportion of pharmaceutical compound 2 in the mixture.

The experimental ED30 is obtained from the combination's DRC, and this value is placed in a Cartesian coordinates system containing the line connecting the ED30 of pharmaceutical compound 1 in the X-axis with the ED30 of pharmaceutical compound 2 in the Y-axis (simple additivity line). The interaction type is determined by the graph region where the experimental value is located in relation to the theoretical value. If the value lies below the additivity line and is statistically different from the theoretical value, then the interaction is synergic (an effect greater than the sum of effects of each pharmaceutical compound); if the value lies near the additivity line and is not statistically different from the theoretical value, then the interaction is only additive (an effect equal to the sum of effects of each pharmaceutical compound). If the value lies in the additivity line and is statistically different from the theoretical value, then the interaction is subadditive or antagonistic. A software calculates the interaction index (I.I.) of two pharmaceutical compounds, obtained with the following formula:
I.I. = Experimental ED30 / theoretical ED30

If the index is less than 1.0, then the interaction is synergic; if it is equal to 1.0, the interaction is additive, and if it is greater than 1.0, the interaction is antagonistic.

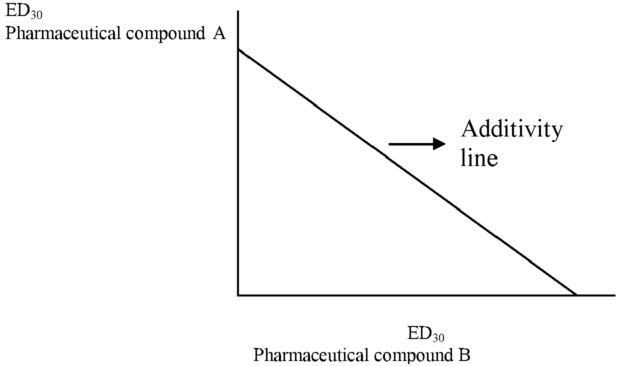

### Experimental sequence

For twelve hours before the experimental preclinical phase, rats fasted but had free access to water.

In order to build the DRC of aceclofenac through intramuscular (IM) administration (10.0, 31.6, 56.2 and 100.0 mg/kg i.m.), the pharmaceutical compound was injected 60 minutes before the formalin injection. For the DRC of betamethasone through intramuscular (IM) administration (0.1, 0.31, 1.0 and 3.1 mg/kg i.m.), the pharmaceutical compound was also injected 60 minutes before the formalin injection.

After obtaining the ED30 of each compound, and for the purpose of performing the isobologram analysis of the aceclofenac and betamethasone combination, the combination ratio was tentatively set at 0.5.

### Data analysis and statistics

The results are expressed as the average ± standard error (SE) of at least 6 rats per group. The number of hind paw shakes/minute was graphed vs. time. The area under the curve (AUC) of temporal courses is a global expression of the effect intensity and duration, and was calculated with the trapezoid method.

The antinoception percentage (%) was calculated with the following formula:
% Antinociception=((Vehicle-Compound)/Vehicle) X 100.

The AUC's and % antinociception were evaluated by means of an analysis of variance (ANOVA) and a Tukey's test. Results with P < 0.05 were considered statistically significant.

### Results

The formalin intraplantar injection caused the typical pain behavior characterized by a brisk withdrawal or bending of the injected hind paw. When the number of shakes was plotted vs. time, the graphs obtained showed the model's distinctive biphasic behavior.

The intramuscular administration of aceclofenac decreased in a dose-dependent way the number of shakes in phase 2 of the formalin test, expressed as % antinociception, and reached a significant difference at a dose of 17.7 mg/Kg IM, and a maximal antinociception effect at a dose of 31.6 mg/Kg IM in both phases. As shown in Figure 2, the ED30 is 13.472 mg/Kg IM.

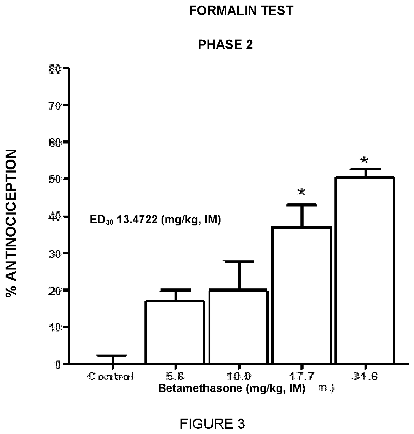

On the other hand, the intramuscular administration of betamethasone decreased in a dose-dependent way the number of shakes in phase 2 of the formalin test, expressed as % antinociception, and reached a significant difference at a dose of 17.7 mg/Kg IM, and a maximal antinociception effect at a dose of 31.6 mg/Kg ilM in both phases. As shown in Figure 3, the ED30 is 13.472 mg/Kg IM.

ED30's and their respective standard errors were estimated from the dose-% effect (% antinociception) curves obtained after the intramuscular administration of aceclofenac and betamethasone.

ED30's and their standard errors estimated after the oral administration of increasing aceclofenac and betamethasone doses are as follows:

| Active principle: | ED30 (mg/Kg, IM) | SE ED30 |
|---|---|---|
| Betamethasone | 13.4722 | 3.1244 |
| Aceclofenac | 1.8662 | 0.1605 |

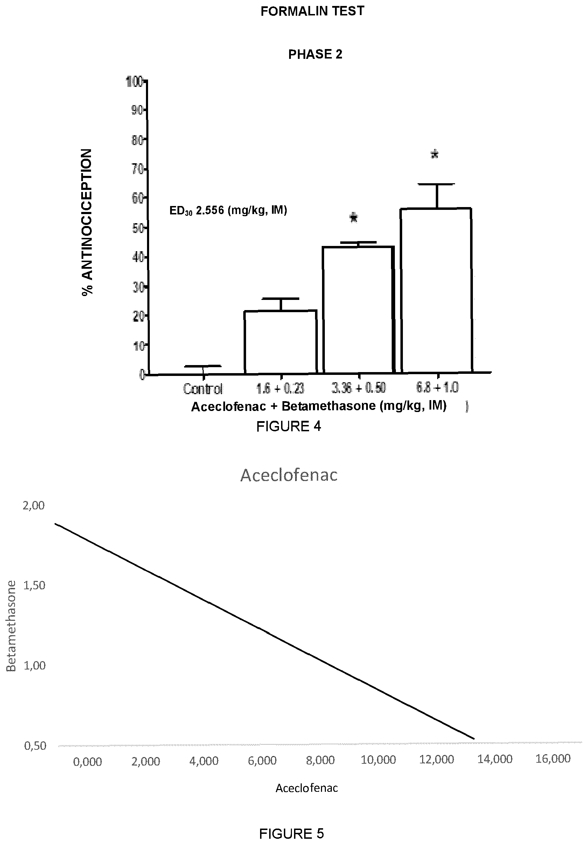

The IM co-administration of the aceclofenac-betamethasone mixture was associated to the doses shown in Figure 4, based on the ED30 values of both pharmaceutical compounds.

Based on the isobologram analysis of the aceclofenac-betamethasone mixture IM administration, it was determined that the interaction of both compounds is synergic, according to the results of the following table, shown graphically in Figure 5.

| | | |
|---|---|---|
| ED30 (mg/Kg, IM) in the experimental | ED30 (mg/Kg, IM) in the theoretical combination | Interaction index |
| combination | | |
| 2.556±0.246 | 7.669±1.564 | 0.333±0.075 |

The isobologram analysis proved the aceclofenac-betamethasone combination's synergy, with an interaction index (I.I.) = 0.333 in the intramuscular administration. It should be kept in mind that an I.I. less than 1.0 implies a synergy of the components, and reflects the reduction of doses needed to reach the test effect of 30% of the maximal effect. It should also be noted that the I.I. observed in the intramuscular combination was the same, since there were no statistical differences between these values. It should also be considered that it is a pain model with an acute inflammatory substrate, and a better effect was observed in this stage (phase 2).

This may imply fewer undesirable effects in the clinical practice, or greater clinical safety in processes requiring short-term prescription of these compounds.

The method allowed to determine the type of interaction resulting when combining aceclofenac and betamethasone, two pharmaceutical compounds having the same pharmacological effect. In this method, less animals were used in the preclinical experimental trial, and less experimentation time was consumed. This technique made possible to compare equieffective doses of the pharmaceutical compounds, when administered both alone and combined. Hence, the administration of aceclofenac with betamethasone did potentiate the antinociceptive effects of both pharmaceutical compounds.

It should be noted that the dose-response curves of aceclofenac with betamethasone 0.05 mg/Kg IM (Figure 6) and aceclofenac with betamethasone 0.5 mg/Kg IM (Figure 7) showed a dose-dependent improved antinociceptive or analgesic effect of aceclofenac. It was also proved that a 0.5 mg/Kg IM dose significantly increased the antinociceptive effect of aceclofenac when compared to a 0.05 mg/Kg IM dose. Thus, combinations were detected that require much lower doses of each compound (in some cases even 30 times less), resulting in significant potentiation effects.

This invention was developed to be administered by intramuscular injection and/or intravenous injection, with lower doses, higher therapeutic power and less risk of adverse events.

### EXAMPLES

The following pharmaceutical compositions are disclosed below as nonlimiting examples:

**Example 1: Intramuscular and intravenous administration compositions.**

| |
|---|
| Aceclofenac |
| Betamethasone or pharmaceutically acceptable phosphate or dipropionate salts thereof |
| Pharmaceutically acceptable excipient and/or carrier |

This invention can be represented in other specific ways without departing from its spirit or essential characteristics. The disclosed embodiments will be in any aspect considered as illustrative and not as limitations. Accordingly, the scope of this invention is defined by the appended claims and not by the above description. Its scope encompasses all modifications within the meaning and range of equivalence of the claims.

In an integral way, this invention provides the following advantages:
1. The intramuscular administration of aceclofenac and betamethasone produces a dose-dependent antinociceptive effect in the second phase of the formalin test in rats.
2. The isobologram analysis proved that the present injectable combination potentiates an antinociceptive effect.
3. The present intravenous or intramuscular injectable combination is useful in the therapy of patients with rheumatic illnesses or post-surgical pain.

## Claims

1. An intramuscular or intravenous injectable pharmaceutical composition **characterized in that** it comprises the synergic combination of:
i. an NSAID agent and/or pharmaceutically acceptable salts thereof,
ii. a corticosteroid agent and/or pharmaceutically acceptable salts thereof,
iii. a pharmaceutically acceptable carrier and/or excipient,
indicated for the control and treatment of rheumatic pain diseases or post-surgical pain in mammals, wherein the NSAID is preferably aceclofenac in base form and the corticosteroid agent is preferably a phosphate or dipropionate salt of betamethasone.

2. The composition of claim 1, wherein the agent aceclofenac is in a concentration of approximately 0.01 to approximately 10,000 mg, being preferably comprised in the composition in a concentration of 0.01 to 5,000 mg.

3. The composition of claim 1, wherein the active agent betamethasone is the phosphate or dipropionate salt thereof in a concentration of approximately 0.001 to approximately 10,000 mg, being preferably comprised in the composition in a concentration of 0.001 to 1 mg.

4. The combination of claim 1, wherein the mammal is a human or an animal.
